# DEMANDE DE BREVET EUROPEEN

(11) **EP 3 718 530 A1**
(43) Date de publication de la demande: **07.10.2020**
(21) Numéro de dépôt: 20174903.3
(22) Date de dépôt: 22.10.2015
(51) Int. Cl.: A61K 8/99, A61K 8/11, A61K 9/48, A61K 36/52, C12N 1/12, C12P 1/00

(54) **CAPSULE GÉLIFIÉE COMPRENANT UNE CELLULE VÉGÉTALE**

(30) Priorité: 22.10.2014 FR 1460169
(62) Demande divisionnaire de: 15786905.8
(71) Demandeur: Capsum, 13013 Marseille (FR)
(72) Inventeur: DULIEGE, Edouard, 75005 Paris (FR); DELMAS, Thomas, 06600 Antibes (FR); BARDON, Sébastien, 75016 Paris (FR); BIBETTE, Jérôme, 75005 Paris (FR); BREMOND, Nicolas, 75005 Paris (FR); DOMEJEAN, Hugo, 75013 Paris (FR)
(74) Mandataire: Lavoix

(57) **Abrégé**

La présente invention concerne une capsule comprenant :
- une phase interne comprenant au moins une cellule végétale, et
- une phase externe gélifiée, encapsulant totalement ladite phase interne à sa périphérie, ladite phase externe comprenant au moins un tensioactif et au moins un polyélectrolyte à l'état gélifié.

## Description

La présente invention concerne une capsule gélifiée comprenant une cellule végétale, ainsi qu'un procédé de mise en culture de ladite cellule végétale.

À l'heure actuelle, les algues macroscopiques (ou macro-algues) sont soit récoltées dans le milieu naturel, soit cultivées dans des fermes en mer. Les micro-algues sont quant à elles principalement cultivées en *open-pond* (mare à ciel ouvert) ou en bio-réacteur.

Le procédé en *open-pond* présente l'avantage d'être peu coûteux, mais ne permet pas de produire les algues en grande concentration. D'autre part, il nécessite de cultiver des organismes extrémophiles afin d'éviter les contaminations (par le milieu extérieur, puisque, par définition, aucune barrière physique n'existe entre le milieu de culture et l'extérieur). On peut notamment citer parmi ces contaminations, le résultat des déjections animales ou de la mort d'insectes et/ou d'animaux.

Le procédé en bio-réacteur permet d'isoler la culture d'algues du monde extérieur et de concentrer les algues. Dans ce procédé, les cellules végétales sont en suspension libres dans un milieu de culture, on parle aussi de procédé en *bulk.* Ce procédé induit toutefois un coût de production important, essentiellement lié au besoin de brasser continuellement la culture et d'y amener les gaz et la lumière nécessaires à la croissance des organismes. D'autre part, les procédés de récolte sont peu aisés et coûteux (centrifugation, filtration tangentielle, etc.). Enfin, le brassage du milieu de culture rend difficile la culture d'algues fragiles, du fait de la mort cellulaire importante engendrée par ce brassage.

Il existe également un procédé de culture tridimensionnelle de cellules de mammifère, dans lequel lesdites cellules sont encapsulées et croissent au contact de la membrane interne des capsules. Néanmoins, ce type de procédé de culture tridimensionnelle n'est pas adapté à la culture de cellules végétales, en particulier de d'organismes unicellulaires.

Il existe donc un besoin pour un procédé permettant d'améliorer la production des cellules végétales, en particulier des algues, en permettant avantageusement d'augmenter la concentration en cellules végétales.

La présente invention a ainsi pour but de fournir un moyen permettant la prolifération (appelée également ici croissance) et, optionnellement, l'élicitation d'au moins une cellule végétale, de préférence d'au moins une cellule d'algue.

La présente invention concerne une capsule comprenant :
- une phase interne comprenant au moins une cellule végétale, et
- une phase externe gélifiée, encapsulant totalement ladite phase interne à sa périphérie, ladite phase externe comprenant au moins un tensioactif et au moins un polyélectrolyte à l'état gélifié.

La présente invention a également pour objet un procédé de culture de cellules végétales, ainsi qu'un procédé de production de composés d'intérêts produits par lesdites cellules végétales, si nécessaire après élicitation.

Dans le cadre de la présente description, la phase interne désigne également le cœur d'une capsule, et la phase externe gélifiée désigne également son enveloppe gélifiée, aussi appelée membrane. Les capsules de l'invention sont aussi appelées capsules gélifiées.

Selon un mode de réalisation, la capsule de l'invention est une capsule dite « simple », signifiant que le cœur est constitué d'une seule phase. Une capsule « simple » est par exemple une capsule telle que décrite dans la demande internationale WO 2010/063937.

La présente invention concerne également un procédé de préparation des capsules selon l'invention.

Les capsules de l'invention sont typiquement préparées par un procédé comprenant les étapes suivantes :
- la mise en contact d'une première solution comprenant au moins une cellule végétale et d'une deuxième solution liquide comprenant au moins un tensioactif et au moins un polyélectrolyte à l'état liquide,
- la formation d'une goutte double comprenant une phase interne formée de la première solution et une phase externe liquide formée de la deuxième solution liquide,
- l'immersion de la goutte double dans une solution gélifiante contenant un réactif propre à gélifier le polyélectrolyte de la phase externe liquide, ce par quoi on obtient la phase externe gélifiée, et
- la récupération des capsules formées.

Dans le cadre de la présente description, on entend par « goutte double » une goutte constituée d'une phase interne et d'une phase externe liquide, encapsulant totalement ladite phase interne à sa périphérie. La production de ce type de goutte est généralement effectuée par co-extrusion concentrique de deux solutions, selon un mode hydrodynamique de *dripping* ou de *jetting,* tel que décrit dans les demandes WO 2010/063937 et FR2964017.

Lorsque la goutte double entre en contact de la solution gélifiante, le réactif propre à gélifier le polyélectrolyte présent dans la solution gélifiante forme alors des liaisons entre les différentes chaînes de polyélectrolyte présentes dans la phase externe liquide. Le polyélectrolyte à l'état liquide passe alors à l'état gélifié, provoquant ainsi la gélification de la phase externe liquide.

Sans vouloir être lié à une théorie particulière, lors du passage à l'état gélifié du polyélectrolyte, les chaînes individuelles de polyélectrolyte présentes dans la phase externe liquide se raccordent les unes aux autres pour former un réseau réticulé, aussi appelé hydrogel, qui emprisonne de l'eau contenue dans la phase externe.

Une phase externe gélifiée, propre à retenir la phase interne de première solution est ainsi formée. Cette phase externe gélifiée présente une tenue mécanique propre, c'est-à-dire qu'elle est capable d'entourer totalement la phase interne et de retenir la ou les cellules végétales présentes dans cette phase interne pour les retenir au cœur de la capsule gélifiée.

Les capsules selon l'invention séjournent dans la solution gélifiante le temps que la phase externe soit complètement gélifiée. Elles sont ensuite collectées et éventuellement plongées dans une solution aqueuse de rinçage, généralement essentiellement constituée d'eau et/ou de milieu de culture.

La taille des différentes phases formant initialement les gouttes doubles, et *in fine* les capsules, est généralement contrôlée par l'utilisation de deux pousse-seringues indépendants (à l'échelle du laboratoire) ou de deux pompes (à l'échelle industrielle), qui fournissent respectivement la première solution et la deuxième solution liquide mentionnées ci-dessus.

Le débit Q_{I} du pousse-seringue associé à la première solution contrôle le diamètre de la phase interne de la capsule finale obtenue.

Le débit Qo du pousse-seringue associé à la deuxième solution liquide contrôle l'épaisseur de la phase externe gélifiée de la capsule finale obtenue.

Le réglage relatif et indépendant des débits Q_{I} et Q_{O} permet de commander l'épaisseur de la phase externe gélifiée indépendamment du diamètre extérieur de la capsule, et de moduler le rapport volumique entre la phase interne et la phase externe.

Les capsules de l'invention présentent généralement une taille moyenne inférieure à 5 mm, et plus généralement comprise de 50 µm à 3 mm, avantageusement de 100 µm à 1 mm.

Selon un mode de réalisation avantageux, la phase externe gélifiée présente une épaisseur moyenne comprise de 5 µm à 500 µm, de préférence de 7 µm à 100 µm.

Selon un mode de réalisation avantageux, le rapport volumique entre la phase interne et la phase externe est supérieur à 1, et de préférence inférieur à 50.

### Phase interne

La phase interne, aussi appelée cœur des capsules, est généralement constituée d'une composition aqueuse, liquide ou visqueuse, comprenant au moins une cellule végétale.

Par « au moins une cellule végétale », on entend au moins une cellule d'une lignée de cellules végétales.

La phase interne peut comprendre plusieurs cellules végétales, de la même lignée ou de lignées différentes.

Les cellules végétales encapsulées constituent ce que l'on appelle aussi la biomasse encapsulée.

Les cellules végétales se différencient des cellules animales, entre autres, par la présence d'une paroi pecto-cellulosique et la présence de plastes, dont les chloroplastes qui permettent la photosynthèse.

Selon un mode de réalisation, les cellules végétales comprises dans la phase interne sont des cellules végétales unicellulaires.

Selon un mode de réalisation, les capsules de l'invention comprennent au moins une cellule d'algue (aussi appelée cellule algale), de préférence une cellule de micro-algue.

Selon une variante, les capsules de l'invention comprennent plusieurs cellules d'algue, d'une même espèce ou d'espèces différentes.

Les algues sont des êtres vivants capables de photosynthèse dont le cycle de vie se déroule généralement en milieu aquatique. Chez les algues, on trouve des procaryotes (cyanobactéries) ou des eucaryotes (plusieurs ensembles très divers).

Le terme « micro-algues » désigne des algues microscopiques. Ce sont des êtres unicellulaires ou pluricellulaires indifférenciés, photosynthétiques, eucaryotes ou procaryotes.

A titre d'algue utilisable dans les capsules de l'invention, on peut citer une algue verte, une algue rouge ou une algue brune.

Selon un mode de réalisation, il s'agit d'une algue procaryote.

Selon un autre mode de réalisation, il s'agit d'une algue eucaryote.

L'algue est de préférence du genre *Chlamydomonas,* comme *Chlamydomonas reinhardtii,* ou du genre *Peridinium,* comme *Peridinium cinctum.*

D'autres algues convenables à la mise en œuvre de l'invention peuvent être choisies dans le groupe constitué de *Alexandrium minutum, Amphiprora hyalina, Anabaena cylindrica, Arthrospira platensis, Chattonella verruculosa, Chlorella vulgaris, Chlorella protothecoides, Chysochromulina breviturrita, Chrysochromulina kappa, Dunaliella salina, Dunaliella minuta, Emiliania huxleyi* (Haptophyta), *Gymnodinium catenatum, Gymnodinium nagasakiense, Haematococcus pluvialis, Isochrysis galbana, Noctiluca scintillans, Odontella aurita, Oryza sativa, Ostreococcus lucimarinus, Pavlova utheri, Porphyridium cruentum, Spirodela oligorrhiza, Spirulina maxima, Tetraselmis tetrathele* et *Thalassiosira pseudonana.*

La phase interne est typiquement apte à la survie de la ou des cellules végétales comprise(s) dans ladite phase interne.

De préférence, la phase interne comprend une solution tampon apte à la survie des cellules végétales.

A titre de tampon utilisable, on peut utiliser tout tampon connu en soi pour être adapté à la survie de cellules végétales.

La phase interne a de préférence un pH compris de 5 à 10, plus préférentiellement compris de 6 à 9.

Selon un mode de réalisation particulièrement adapté à la culture de cellules végétales, la phase interne comprend des nutriments aptes à la prolifération de la ou des cellules végétales.

De préférence, la phase interne comprend un milieu de culture appelé MC1 dans le cadre de la présente invention.

Par « milieu de culture », on entend une solution comprenant des nutriments aptes à la prolifération de la ou des cellules végétales et jouant le rôle de tampon pH.

L'osmolarité du milieu de culture MC1 est de préférence comprise entre 10 mOsm et 1 000 mOsm.

Le milieu de culture MC1 est par exemple choisi parmi le milieu de culture d'Erdschreiber, le milieu F/2, le milieu TAP, de l'eau de mer reconstituée, le milieu DM (diatomée), le milieu Minimum, et l'un quelconque de leurs mélanges.

Le milieu de culture d'Erdschreiber est une solution comprenant NaCI (11,4 g/L), Tris (5,90 g/L), NH₄Cl (2,92 g/L), KCI (0,73 g/L), K₂HPO₄.2H₂O (72 mg/L), FeSO₄.2H₂O (1,9 mg/L), H₂SO₄ (0,04 mg/L), MgSO₄ (7,65 g/L), CaCl₂ (1,43 g/L), NaNO₃ (2 mg/L), Na₂HPO₄ (0,2 mg/L), un extrait de sol (24,36 mL/L) et de l'eau (QSP 1 L). Un extrait de sol désigne le filtrat obtenu par filtration d'un mélange de terre et d'eau.

Le milieu F/2, commercialement disponible (notamment à l'Ecole des Sciences Biologiques de l'Université du Texas, ou chez Varicon Aqua), est une solution comprenant NaNO₃ (8,82.10⁻⁴ mol/L), NaH₂PO₄O.H₂O (3,62.10⁻⁵ mol/L), Na₂SiO₃.9H₂O (1,06.10⁻⁴ mol/L), FeCl₃.6H₂O (1,17.10⁻⁵ mol/L), Na₂EDTA.2H₂O (1,17.10⁻⁵ mol/L), CuSO₄.5H₂O (3,93.10⁻⁸ mol/L), Na₂MoO₄.2H₂O (2,60.10⁻⁸ mol/L), ZnSO₄.7H₂O (7,65.10⁻⁸ mol/L), COCl₂.6H₂O (4,20.10⁻⁸ mol/L), MnCl₂.4H₂O (9,10.10⁻⁷ mol/L), thiamine HCI (2,96.10⁻⁷ mol/L), biotine (2,05.10⁻⁹ mol/L), cyanocobalamine (3,69.10⁻¹⁰ mol/L) et de l'eau (QSP 1 L).

Le milieu TAP, commercialement disponible, (notamment chez LifeTech), est un mélange de tampon Beijerincks (2x) (50 mL), de tampon phosphate 1M pH 7 (1 mL), de Solution trace (1 mL), d'acide acétique (1 mL) et d'eau (QSP 1 L). Les compositions des tampons Beijerincks (2x) et phosphate 1M pH 7 et de la Solution trace sont décrites dans les exemples ci-après. Le pH du milieu TAP est de 7,3. L'osmolarité du milieu TAP est de 60 mOsm.

L'eau de mer reconstituée est une solution comprenant NaCI (11,7 g/L), Tris (6,05 g/L), NH₄Cl (3,00 g/L), KCI (0,75 g/L), K₂HPO₄.2H₂O (74,4 mg/L), FeSO₄.2H₂O (2 mg/L), H₂SO₄ (0,05 mg/L), MgSO₄ (7,85 g/L), CaCl₂ (1,47 g/L) et de l'eau (QSP 1 L). Le pH de l'eau de mer reconstituée varie de 7,5 à 8,4. L'osmolarité de l'eau de mer reconstituée est de 676 mOsm.

Le milieu DM (diatomée) est une solution comprenant Ca(NO₃)₂ (20 g/L), KH₂PO₄ (12,4 g/L), MgSO₄.7H₂O (25 g/L), NaHCO₃ (15,9 g/L), FeNaEDTA (2,25 g/L), Na₂EDTA (2,25 g/L), H₃BO₃ (2,48 g/L), MnCl₂.4H₂O (1,39 g/L), (NH₄)₆Mo₇O₂₄.4H₂O (1 g/L), cyanocobalamine (0,04 g/L), thiamine HCI (0,04 g/L), biotine (0,04 g/L), NaSiO₃.9H₂O (57 g/L) et de l'eau (QSP 1 L).

Le milieu Minimum est un mélange de tampon Beijerincks (2x) (50 mL), de tampon phosphate (2x) (50 mL), de Solution trace (1 mL) et d'eau (QSP 1 L). Les compositions des tampons Beijerincks (2x) et phosphate (2x) et de la Solution trace sont décrites dans les exemples ci-après. L'osmolarité du milieu Minimum est de 37 mOsm.

Lors de l'encapsulation des cellules végétales (i.e. avant tout procédé de culture des cellules végétales), la phase interne comprend typiquement de 10³ à 10⁹, de préférence de 10⁴ à 10⁸, plus préférentiellement de 10⁵ à 10⁸, par exemple de 10⁶ à 5.10⁶ cellules végétales, par millilitre de phase interne.

En fonction de la taille des capsules, la phase interne comprend typiquement de 1 à 10⁷, préférentiellement de 5 à 10⁶, de 30 à 5.10⁵, de 50 à 10⁵, de 75 à 5.10⁴, de 100 à 10⁴, de 150 à 10⁴, voire de 200 à 10³ cellules végétales, par capsule.

Le comptage des cellules végétales de la phase interne est préférentiellement réalisé avant l'encapsulation, ou bien après l'ouverture des capsules.

Le comptage des cellules végétales peut être fait par la méthode de comptage Malassez. La cellule de Malassez est une lame de verre qui permet de compter le nombre de cellules en suspension dans une solution. Sur cette lame de verre, un quadrillage de 25 rectangles a été gravé, contenant eux-mêmes 20 petits carrés. Pour dénombrer les cellules végétales, on dépose sur la cellule de Malassez entre 10 µL et 15 µL de phase interne comprenant des cellules végétales en suspension. Après sédimentation, on compte le nombre de cellules végétales dans 10 rectangles (quadrillés). Le volume d'un rectangle quadrillé étant de 0,01 µL, on multiplie ce nombre par 10 000 pour obtenir le nombre de cellules végétales par millilitre de phase interne.

Alternativement, le comptage des cellules végétales peut être fait par mesure d'absorbance. D'après la loi de Beer-Lambert, pour une longueur d'onde λ donnée, l'absorbance d'une solution est proportionnelle à sa concentration et à la longueur du trajet optique (distance sur laquelle la lumière traverse la solution). On peut donc mesurer la concentration en cellules végétales de la phase interne en se basant sur une méthode de mesure d'absorbance (encore appelée densité optique). Il suffit pour cela de mesurer les densités optiques de phases internes contenant une quantité connue de cellules végétales, ce qui permet de construire une courbe-étalon en fonction de la concentration cellulaire.

A titre d'exemple, la phase interne comprend un million de cellules par millilitre de phase interne avant tout procédé de culture, ce qui correspond à environ 60 cellules par capsule de 500 µm de diamètre.

Après un procédé de culture tel que décrit ci-après, la phase interne comprend typiquement de 50 millions à 150 millions de cellules végétales, par millilitre de phase interne.

Avantageusement, les cellules végétales présentes dans la phase interne des capsules sont en suspension dans la phase interne.

Par « en suspension » dans la phase interne, on entend que les cellules végétales n'adhèrent pas à la membrane gélifiée des capsules, et ne sont pas en contact prolongé avec ladite membrane. Les cellules végétales sont ainsi totalement baignées dans le milieu constituant la phase interne et sont libres de se déplacer selon les trois dimensions.

L'homme du métier est capable de vérifier que les cellules végétales sont effectivement en suspension dans les capsules, typiquement en observant les capsules par microscopie et en mettant en évidence un mouvement différencié entre la capsule et les cellules végétales qu'elle contient. Par exemple, dans le cas particulier des micro-algues flagellées, on peut observer leur nage, due à l'action de leurs flagelles.

Selon un mode de réalisation, la phase interne comprend au moins un agent de viscosité, de préférence biocompatible, typiquement choisi parmi les éthers de cellulose.

La présence d'un agent de viscosité permet de faciliter la préparation des capsules en diminuant la différence de viscosité entre la phase interne et la phase externe, qui comprend un polyélectrolyte en solution.

Par « agent de viscosité », on entend un produit soluble dans la phase interne apte à moduler sa viscosité. Il peut notamment s'agir d'un polymère naturel, comme les glycosaminoglycanes (acide hyaluronique, chitosan, héparane sulfate, etc), l'amidon, les protéines de plantes, la gomme welan, ou toute autre gomme naturelle ; d'un polymère semi-synthétique, comme les amidons décomposés et leurs dérivés, les éthers de cellulose, comme l'hydroxypropyl méthyl cellulose (HPMC), l'hydroxy ethyl cellulose (HEC), le carboxy méthyl cellulose (CMC) et le 2-ethylcellulose ; ou encore d'un polymère synthétique, comme les polyéthers (polyéthylène glycol), les polyacrylamides, et les polyvinyles.

De préférence, la phase interne comprend un éther de cellulose, comme le 2-ethylcellulose.

Lorsqu'il est présent, l'agent de viscosité est présent dans la phase interne selon une concentration massique de 0,01% à 5%, préférentiellement de 0,1% à 1%, par rapport à la masse totale de la phase interne.

### Phase externe

La phase externe comprend au moins un polyélectrolyte à l'état gélifié, aussi appelé polyélectrolyte gélifié, et au moins un tensioactif.

### Polyélectrolyte

De préférence, le polyélectrolyte est choisi parmi les polyélectrolytes réactifs aux ions multivalents.

Dans le cadre de la présente description, on entend par « polyélectrolyte réactif aux ions multivalents » un polyélectrolyte susceptible de passer d'un état liquide dans une solution aqueuse à un état gélifié sous l'effet d'un contact avec une solution gélifiante contenant des ions multivalents, tels que des cations multivalents de calcium, baryum, magnésium, aluminium ou fer.

A l'état liquide, les chaînes individuelles de polyélectrolyte sont sensiblement libres de s'écouler les unes par rapport aux autres. Une solution aqueuse de 2% en masse de polyélectrolyte présente alors un comportement purement visqueux aux gradients de cisaillement caractéristiques du procédé de mise en forme. La viscosité de cette solution à cisaillement nul est entre 50 mPa.s et 10 000 mPa.s, avantageusement entre 1 000 mPa.s et 7 000 mPa.s.

Les chaînes individuelles de polyélectrolyte à l'état liquide présentent avantageusement une masse molaire supérieure à 65 000 g/mol.

La solution gélifiante est par exemple une solution aqueuse d'un sel de formule XₙMₘ, où :
- X est choisi parmi le groupe constitué des ions halogénures (chlorure, bromure, iodure et fluorure) et des ions tartrate, lactate et carbonate,
- M est choisi parmi le groupe constitué des cations Ca²⁺, Mg²⁺, Ba²⁺, Al³⁺ et Fe³⁺, et
- n et m sont supérieurs ou égaux à 1.

La concentration en sel XₙMₘ dans la solution gélifiante est avantageusement comprise de 1% à 20% en masse, préférentiellement de 5% à 20% en masse.

A l'état gélifié, les chaînes individuelles de polyélectrolyte forment, avec les ions multivalents, un réseau tridimensionnel cohérent qui retient la phase interne et empêche son écoulement. Les chaînes individuelles sont retenues les unes par rapport aux autres et ne peuvent s'écouler librement les unes par rapport aux autres.

Le polyélectrolyte est de préférence un polymère biocompatible, il est par exemple produit biologiquement.

Avantageusement, il est choisi parmi les polysaccharides, les polyélectrolytes de synthèse à base d'acrylates (polyacrylate de sodium, de lithium, de potassium ou d'ammonium, ou polyacrylamide), ou les polyélectrolytes de synthèse à base de sulfonates (poly(styrène sulfonate) de sodium, par exemple).

Plus particulièrement, le polyélectrolyte est choisi parmi les alginates d'alcalin, tel qu'un alginate de sodium ou un alginate de potassium, les géllanes et les pectines.

Dans le cas où le polyélectrolyte est un alginate de sodium (NaAlg), et où le réactif est le chlorure de calcium, la réaction qui se produit lors de la gélification est la suivante :

2NaAlg + CaCl₂ → Ca(Alg)₂ + 2NaCl

Les alginates sont produits à partir d'algues brunes appelées « laminaires », désignées par le terme anglais « sea weed ».

De préférence, le polyélectrolyte est un alginate d'alcalin ayant avantageusement une teneur en bloc α-L-guluronate supérieure à 50%, notamment supérieure à 55%, voire supérieure à 60%.

Le polyélectrolyte est par exemple un alginate de sodium.

Le polyélectrolyte à l'état gélifié est typiquement un alginate de calcium.

Selon un mode de réalisation préféré, le pourcentage massique total en polyélectrolyte dans la phase externe gélifiée est compris de 0,5% à 5%, de préférence inférieur à 3%.

Le pourcentage massique total en polyélectrolyte dans la phase externe gélifiée est par exemple compris entre 0,5% et 3%, de préférence entre 1% et 2%.

### Tensioactif

La présence d'un tensioactif dans la phase externe permet de faciliter la préparation des capsules de l'invention, en augmentant la résistance de la phase externe liquide lors de l'impact de la goutte double avec la solution gélifiante.

Le tensioactif est avantageusement un tensioactif anionique, un tensioactif non ionique, un tensioactif cationique ou un mélange quelconque de ceux-ci. La masse moléculaire de l'agent tensioactif est typiquement comprise entre 150 g/mol et 10 000 g/mol, avantageusement entre 250 g/mol et 1 500 g/mol.

De manière générale, la teneur massique en tensioactif dans la phase externe est typiquement inférieure ou égale à 2%, de préférence inférieure à 1%, par rapport à la masse totale de la capsule.

Dans le cas où le tensioactif est un tensioactif anionique, il est par exemple choisi parmi les alkylsulfates, les alkylsulfonates, les alkylarylsulfonates, les alkylphosphates alcalins, les dialkylsulfosuccinates, les sels d'alcalino-terreux d'acides gras saturés ou non. Ces tensioactifs présentent avantageusement au moins une chaîne hydrocarbonée hydrophobe présentant un nombre de carbones supérieur à 5, voire 10 et au moins un groupement anionique hydrophile, tel qu'un sulfate, un sulfonate ou un carboxylate lié à une extrémité de la chaîne hydrophobe.

Un tensioactif anionique particulièrement approprié à la mise en œuvre de l'invention est le dodécylsulfate de sodium (SDS).

Lorsque le tensioactif est un tensioactif anionique, la teneur massique en tensioactif dans la phase externe est typiquement comprise de 0,001% à 0,5%, de préférence de 0,001% à 0,05%, par rapport à la masse totale de la capsule.

Dans ce cas, la teneur massique en tensioactif dans la phase externe est de préférence inférieure ou égale à 0,025%, préférentiellement inférieure ou égale à 0,010%, voire inférieure ou égale à 0,005%, par rapport à la masse totale de la capsule.

Dans le cas où le tensioactif est un tensioactif cationique, il est par exemple choisi parmi les sels d'halogénures d'alkylpyridium ou d'alkylammonium comme le chlorure ou le bromure de n-éthyldodécylammonium, le chlorure ou le bromure de cétylammonium (CTAB). Ces tensioactifs présentent avantageusement au moins une chaîne hydrocarbonée hydrophobe présentant un nombre d'atomes de carbone supérieur à 5, voire 10 et au moins un groupement cationique hydrophile, tel qu'un cation d'ammonium quaternaire.

Lorsque le tensioactif est un tensioactif cationique, la teneur massique en tensioactif dans la phase externe est typiquement comprise de 0,001% à 0,5%, de préférence de 0,001% à 0,05%, par rapport à la masse totale de la capsule.

Dans ce cas, la teneur massique en tensioactif dans la phase externe est de préférence inférieure ou égale à 0,025%, préférentiellement inférieure ou égale à 0,010%, voire inférieure ou égale à 0,005%, par rapport à la masse totale de la capsule.

Dans le cas où le tensioactif est un tensioactif non ionique, il est par exemple choisi parmi des dérivés polyoxyéthylénés et/ou polyoxypropylénés des alcools gras, des acides gras, ou des alkylphénols, des arylphénols, ou parmi des alkylglucosides, des polysorbates et des cocamides.

Un tensioactif non ionique particulièrement approprié à la mise en œuvre de l'invention est le polysorbate 20 (Tween 20).

Lorsque le tensioactif est un tensioactif non ionique, la teneur massique en tensioactif dans la phase externe est typiquement comprise de 0,01% à 2%, de préférence de 0,1% à 1%, par rapport à la masse totale de la capsule.

### Phase intermédiaire

Selon un mode de réalisation, les capsules selon l'invention comprennent une phase intermédiaire entre la phase interne et la phase externe gélifiée.

Cette phase intermédiaire forme une enveloppe intermédiaire aqueuse, ou le cas échéant huileuse, généralement biocompatible, qui encapsule totalement la phase interne et est totalement encapsulée par la phase externe gélifiée.

De telles capsules sont généralement obtenues par co-extrusion concentrique de trois solutions, au moyen d'une triple enveloppe : un premier flux constitue la phase interne, un deuxième flux constitue la phase intermédiaire et un troisième flux constitue la phase externe. La production de telles capsules, dites « complexes », est notamment décrite dans la demande internationale WO 2012/089820.

A la sortie de la triple enveloppe, les trois flux entrent en contact et se forme alors une goutte multi-composante, qui est ensuite gélifiée lorsqu'elle est plongée dans une solution gélifiante, de la même manière que dans le procédé de préparation de capsules « simples » décrit ci-dessus.

La phase intermédiaire peut comprendre au moins une cellule végétale, de préférence une cellule d'algue, qui peut être identique ou différente des cellules végétales présentes dans la phase interne.

La phase intermédiaire peut aussi comprendre au moins un agent de viscosité tel que décrit ci-dessus.

La phase intermédiaire, lorsqu'elle est présente et qu'elle comprend des cellules végétales, est de préférence apte à la survie desdites cellules végétales. Elle comprend avantageusement un milieu de culture apte à la culture desdites cellules, typiquement un des milieux de culture MC1 mentionnés ci-dessus pour la phase interne.

La phase intermédiaire, lorsqu'elle est présente et qu'elle comprend des cellules végétales, comprend typiquement de 1 à 10⁷, préférentiellement de 5 à 10⁶, de 30 à 5.10⁵, de 50 à 10⁵, de 75 à 5.10⁴, de 100 à 10⁴, de 150 à 10⁴, voire de 200 à 10³ cellules végétales, par capsule.

Selon un mode de réalisation, la capsule selon l'invention est constituée :
- d'une phase interne comprenant au moins une cellule végétale, et
- d'une phase externe gélifiée, encapsulant totalement ladite phase interne à sa périphérie, ladite phase externe comprenant au moins un tensioactif et au moins un polyélectrolyte à l'état gélifié.

Selon un mode de réalisation, la capsule selon l'invention ne comprend pas d'enveloppe (ou encore membrane) autre que la phase interne et l'enveloppe gélifiée (phase externe gélifiée). En particulier, la capsule selon l'invention ne comprend pas d'enveloppe rigidifiée telle que celle décrite dans FR 2 986 165 ou dans WO 2013/113855.

L'enveloppe rigidifiée de FR 2 986 165 et WO 2013/113855 permet notamment la fixation et le développement de cellules de mammifères.

Ainsi, selon un mode de réalisation préféré, la capsule selon l'invention est dénuée d'enveloppe rigidifiée, et notamment d'enveloppe intermédiaire rigidifiée.

### Procédé de culture

La présente invention a également pour objet un procédé de culture de cellules végétales comprenant une étape de mise en culture d'au moins une capsule selon l'invention.

Par « mise en culture », on entend l'action de placer les capsules de l'invention dans un milieu de culture appelé MC2 dans le cadre de la présente invention, typiquement apte à la culture des cellules végétales encapsulées, dans des conditions de température et de luminosité adaptées à la culture desdites cellules végétales, pendant une durée nécessaire pour obtenir la concentration en cellules végétales souhaitées au sein des capsules.

En fonction des cellules végétales mise en culture, l'homme du métier est apte à sélectionner le milieu de culture MC2 approprié, ainsi que les conditions de température et de luminosité appropriées à la prolifération des cellules végétales.

Le milieu de culture MC2 est par exemple choisi parmi le milieu de culture d'Erdschreiber, le milieu F/2, le milieu TAP, de l'eau de mer reconstituée, le milieu DM (diatomée), le milieu Minimum, et l'un quelconque de leurs mélanges.

L'osmolarité du milieu de culture MC2 est de préférence comprise entre 10 mOsm et 1 000 mOsm.

Il est préférable que le ratio (osmolarité de MC1)/(osmolarité de MC2) soit compris entre 1/50 et 50, préférentiellement entre 1/10 et 10.

Selon un mode de réalisation, le milieu de culture MC2 est identique au milieu de culture MC1.

Selon un autre mode de réalisation, le milieu de culture MC2 est différent du milieu de culture MC1.

Il est avantageux de choisir des milieux de culture différents pour provoquer la production de composés d'intérêt au sein des cellules simultanément à la mise en culture.

Typiquement, pour la mise en culture, on place de 10 000 à 10 000 000 capsules selon l'invention par litre de milieu de culture MC2.

Les capsules sont typiquement mises en culture à une température comprise de 10°C à 40°C, préférentiellement de 15°C à 25°C.

Les capsules sont typiquement mises en culture dans des conditions de luminosité allant du noir total à 500 µE.m⁻².s⁻¹.

Les capsules sont typiquement mises en culture pendant une durée allant de une heure à un mois, généralement de 24 heures à une semaine.

A la fin du procédé de culture, la récolte des capsules se fait typiquement par élimination du milieu de culture MC2 par filtration des capsules, ou par toute autre technique de récupération des capsules.

Pour filtrer les capsules, on utilise typiquement un tamis présentant une taille d'ouverture inférieure au diamètre moyen des capsules de l'invention, qui sont sensiblement sphériques.

Les inventeurs ont découvert, de manière surprenante, que la culture de cellules végétales, en particulier d'algues, au sein des capsules selon l'invention permet d'accéder à des concentrations de cellules végétales plus élevées que dans les procédés en *bulk* mentionnés en introduction. Par exemple, des concentrations cellulaires de 50 millions à 150 millions de cellules par mL sont obtenues dans les capsules, soit de 5 à 15 fois plus que celles obtenues dans des procédés en *bulk.*

L'encapsulation de cellules végétales et la culture en capsules de ces cellules végétales, en particulier d'algues telles que des micro-algues, présente en outre les avantages suivants par rapport aux procédés classiques :
- les cellules végétales encapsulées sont protégées des contraintes mécaniques, telles que le cisaillement, diminuant ainsi la mort cellulaire au cours du procédé de culture des cellules ;
- les cellules végétales encapsulées sont partiellement protégées du milieu extérieur, car la membrane de la capsule présente une perméabilité sélective (en particulier, les bactéries ne peuvent pas pénétrer dans la capsule), ce qui permet avantageusement d'éviter d'éventuelles contaminations et donc de réduire la mort cellulaire ;
- la taille des capsules (plusieurs centaines de micromètres) et leurs propriétés mécaniques les rendent plus faciles à manipuler que les algues, notamment pour des changements de milieu de culture MC2 ou lors de la récolte ; et
- un traitement postérieur à la croissance et éventuellement à l'élicitation peut être mis en œuvre afin d'imperméabiliser les capsules, isolant ainsi leur contenu pour en faciliter la conservation jusqu'à leur utilisation.

Certains avantages de l'invention dus aux propriétés mécaniques de l'hydrogel formant la membrane des capsules vont maintenant être détaillés.

Le réseau d'hydrogel confère en effet des propriétés mécaniques semi-solides aux capsules de l'invention, bien supérieures à celles de cellules végétales.

En pratique, cela présente un intérêt significatif pour la culture de cellules végétales, tels que les algues, car cela permet de conférer une résistance mécanique importante à des cellules végétales, classiquement fragiles. Alors que la résistance mécanique de ces cellules végétales repose nativement sur les propriétés de la bicouche lipidique (3 nm de phospholipides) constituant leur membrane, lorsqu'elles sont encapsulées dans une capsule selon l'invention, celles-ci sont désormais protégées par un réseau macromoléculaire de plusieurs micromètres à plusieurs dizaines de micromètres d'épaisseur (i.e. la membrane d'hydrogel des capsules). Dans le cas de cellules végétales ayant une résistance mécanique faible, un gain de résistance d'un facteur supérieur à 10, voire compris de 100 à 100 000, est obtenu, ce qui facilite les manipulations.

On obtient ainsi des objets « solides » plus facilement manipulables, à l'échelle de procédés industriels de type mélange, brassage, filtrage, lavage et décantage.

Il est également plus facile de manipuler ces objets, et donc la biomasse qu'ils encapsulent, pour faciliter la caractérisation différentielle des échantillons. Ceci permet la mise en œuvre facilitée et économique de larges études de *screening* de conditions de culture et/ou d'élicitation de composés d'intérêt. Suite à une caractérisation de chacune des capsules par voie physicochimique (absorbance, fluorescence) et/ou biologique (ELISA), on réalise par exemple un traitement statistique, ou un tri des différentes populations en fonction de la réponse obtenue.

La membrane d'hydrogel des capsules de l'invention présente également une semi-perméabilité, reposant sur le caractère poreux de la structure du réseau de chaînes du polyélectrolyte. Ce réseau est caractérisé par une taille moyenne de pores comprise entre 5 nm et 25 nm, et présente donc une taille de coupure de l'ordre de 20 nm à 25 nm. Cette porosité permet le libre passage de gaz dissous, de minéraux, de nutriments nécessaires à la prolifération de cellules végétales, tels que de petites biomolécules (acides aminés et peptides), et de petites macromolécules de poids moléculaire inférieur à 1 MDa. La membrane retient par contre tout élément de taille caractéristique supérieure à la taille de coupure, soit les macromolécules ou biomolécules de poids moléculaire supérieur à 1 MDa, ou encore les organismes cellulaires tels que les cellules végétales, par exemple les algues, ou encore les bactéries et les champignons.

En pratique, cela présente un intérêt significatif pour la culture de cellules végétales, comme les algues, en permettant de contrôler les échanges entre l'extérieur et l'intérieur de la capsule, c'est-à-dire entre le milieu de culture MC2 et la biomasse encapsulée.

Il est ainsi possible d'apporter des nutriments, de l'oxygène ou encore de la lumière à la biomasse encapsulée par transit à travers la phase externe. Il est parallèlement possible de permettre la sortie de molécules depuis l'intérieur de la capsule vers le milieu de culture MC2, notamment pour l'élimination des déchets consécutifs du métabolisme/catabolisme de la biomasse encapsulée. Ces échanges peuvent de plus être facilités par un brassage du milieu de culture MC2, sans que cela ne menace les cellules végétales comme c'est le cas dans les procédés en *bulk.*

Il est également possible de limiter le passage d'organismes cellulaires à travers la membrane. Il est donc possible d'encapsuler les cellules végétales avec l'ensemble de la flore bactérienne endogène apte à leur prolifération, tout en évitant toute contamination par des bactéries exogènes après l'encapsulation. Il est par exemple possible de laver les capsules contenant les cellules végétales après une contamination bactérienne exogène, là où la même contamination engendrerait la destruction de l'ensemble des cellules dans un procédé en *bulk.*

Enfin, cette semi-perméabilité permet également de contrôler le relargage d'élément encapsulé, tel que des composés d'intérêt produits par les cellules végétales.

La production des cellules végétales, en particulier d'algues, sous forme encapsulée conformément à l'invention permet aussi de limiter les effets de détection du quorum (*quorum sensing*).

Lors de leur croissance, les organismes libèrent généralement des molécules inhibitrices, qui limitent la prolifération des organismes alentours. Cet effet biologique permet aux populations de limiter leur densité, afin d'éviter les effets de privation de nourriture et de mort cellulaire associés à une surpopulation. L'encapsulation permet de drainer en continu ces molécules inhibitrices, sans entraîner de perte de biomasse.

Un lavage est aussi utile pour éliminer des déchets métaboliques ou cataboliques, et orienter ainsi l'activité de la biomasse. Cette approche se révèle ainsi être une méthode d'élicitation en soi.

Par ailleurs, un des facteurs limitant de la culture d'algues est l'apport de lumière. Dans un procédé en *bulk,* le flux de lumière est diminué par le « *fouling* », c'est-à-dire le collage de biomolécules et de micro-organismes sur les parois du bioréacteur. Cette couche absorbe la lumière, ce qui induit une baisse du flux lumineux capté par les cellules végétales. L'accumulation de ce dépôt induit des pertes de charge croissantes et augmente les coûts de production.

Or, la culture de cellules végétales en capsules selon l'invention permet de :
- limiter le dépôt des biomolécules, celles-ci étant en partie retenues à l'intérieur des capsules, de plus ces biomolécules peuvent être récupérées en fin de procédé, et
- empêcher le dépôt des cellules végétales sur les parois des flacons dans lesquels les capsules sont cultivées ou stockées (propriété anti-fouling), lesdites cellules étant en effet encapsulées.

Enfin, ce lavage peut aussi être envisagé pour éliminer de possibles contaminants de la culture de biomasse, sans manipulation directe de celle-ci. Ces contaminants peuvent être d'origine chimique (molécules de type métaux lourds ou autres rejets chimiques), physique (de type particulaire), ou biologique (cellules mortes, bactéries exogènes...). Cette méthode peut donc permettre de limiter les pertes d'exploitation.

### Procédé de production d'un composé d'intérêt

La présente invention a également pour objet un procédé de production d'un composé d'intérêt, comprenant :
- une étape de mise en culture d'au moins une capsule selon l'invention, ladite capsule comprenant au moins une cellule végétale apte à produire ledit composé d'intérêt,
- éventuellement, une étape d'élicitation des cellules végétales comprises dans ladite capsule, et
- une étape de récupération du composé d'intérêt.

L'étape de mise en culture du procédé de production ci-dessus correspond généralement à l'étape de mise en culture du procédé de culture de l'invention. Le milieu de culture MC2 peut être identique ou différent du milieu de culture MC1 du cœur des capsules.

Dans le cadre de la présente description, on entend par « élicitation » la stimulation de la production de composés d'intérêt par une cellule végétale, ladite stimulation étant provoquée par la mise en conditions particulières, qu'elles soient physico-chimiques, résultant d'une modulation de la température, de la pression ou de l'éclairement, ou encore qu'elles reposent sur la présence d'une molécule particulière, dite « molécule élicitante ». On provoque ainsi artificiellement la production de composés d'intérêt par les cellules végétales encapsulées.

Selon un mode de réalisation, l'étape d'élicitation a lieu pendant l'étape de mise en culture.

Ce mode de réalisation est par exemple mis en œuvre en effectuant l'étape de mise en culture dans des conditions élicitantes, par exemple en effectuant l'étape de mise en culture dans un milieu de culture MC2 différent du milieu de culture MC1, ledit milieu de culture MC2 contenant une molécule élicitante.

Selon un autre mode de réalisation, l'étape d'élicitation a lieu à l'issue de l'étape de mise en culture, c'est-à-dire une fois que les cellules végétales ont proliféré au sein des capsules de l'invention.

Ce mode de réalisation est par exemple mis en œuvre en effectuant l'étape de mise en culture dans des conditions classiques, c'est-à-dire en choisissant un milieu de culture MC2 identique au milieu de culture MC1, puis, à l'issue de l'étape de mise en culture, en se plaçant dans des conditions élicitantes, par exemple en remplaçant le milieu de culture MC2 par un autre milieu de culture, différent du milieu de culture MC1.

Ces deux modes de réalisation sont facilités par l'encapsulation des cellules végétales, qui rend les manipulations des cellules encapsulées plus pratiques. Comme décrit plus haut, du fait de l'encapsulation, il est en effet plus facile de faire proliférer les cellules végétales au sein des capsules, puis éventuellement de diviser les capsules en lots séparés, de remplacer le milieu de culture MC2 par divers milieux de culture et/ou d'appliquer divers conditions d'élicitation en fonction des lots, ce afin de tester différentes conditions élicitantes et de déterminer les conditions adéquates à la production du composé d'intérêt que l'homme du métier souhaite obtenir.

Ainsi, après une première étape de mise en culture, l'étape d'élicitation des cellules végétales comprend typiquement :
- une étape de mise en culture des capsules en lots séparés dans des conditions différentes,
- une étape de détection et de quantification du composé d'intérêt produit, et
- éventuellement la sélection des individus présentant les meilleurs phénotypes.

L'étape d'élicitation des cellules végétales consiste par exemple à mettre en culture les capsules de l'invention dans un milieu de culture MC2 différent du milieu de culture MC1, ou dans un milieu de culture comprenant une molécule élicitante, ou dans le même milieu MC1 avec une modification des conditions de culture (température, lumière, etc.).

Les capsules selon l'invention peuvent être mises en culture en conditions élicitantes bien connues de l'homme du métier, comme par l'ajout au milieu de culture MC2 d'acide salicylique, d'éthylène, de jasmonate ou de chitosan (cf. par exemple la demande internationale WO 2003/077881).

Les composés d'intérêt produits par le procédé de production de l'invention sont typiquement soumis à un ou plusieurs traitements, tels que purification, concentration, séchage, stérilisation et/ou extraction. Ces composés sont ensuite destinés à être incorporés dans une composition cosmétique, agro-alimentaire ou pharmaceutique.

A titre de composé d'intérêt, les capsules de l'invention permettent notamment de produire des lipides présentant un intérêt en cosmétique, comme par exemple des acides gras, tels que l'acide linoléique, l'acide alpha linoléique, l'acide gamma linoléique, l'acide palmitique, l'acide stéarique, l'acide eicosapentanoïque, l'acide docosahexanoïque, l'acide arachidonique ; des dérivés d'acide gras, tels que des céramides ; ou encore des stérols, tels que le brassicastérol, le campestériol, le stigmastérol et le sitostérol.

Les capsules de l'invention permettent également de produire du sélénium organique, oligo-élément indispensable.

Cet élément chimique, non synthétisé par le corps humain, se présente comme un actif d'intérêt en agroalimentaire et en cosmétique, notamment pour ses propriétés anti-oxydantes. Sous sa forme métallique, c'est un élément trace essentiel mais nombre de ses composés sont extrêmement toxiques, et sont obtenus par retraitement des résidus de l'électrolyse du plomb, de l'arsenic ou du cuivre. C'est pourquoi il est préférable de l'obtenir sous sa forme organique pour les applications biologiques, c'est-à-dire en tant que constituant de biomolécules.

Le sélénium est typiquement incorporé dans des acides aminés, des peptides et des protéines, notamment sous la forme de sélénométhionine.

Selon la nature du ou des composés d'intérêt à récupérer et celle des cellules végétales, l'homme du métier est apte à déterminer le ou les traitements nécessaires à la récupération et purification dudit composé d'intérêt.

Selon un mode de réalisation, la récupération du composé d'intérêt s'effectue par traitement du milieu de culture (MC2) dans lequel les capsules sont plongées, par des méthodes de purification classiques, comme l'extraction liquide/liquide (séparation de phase organique/aqueuse), le lavage acido-basique, la concentration de solvant et/ou la purification par chromatographie, entre autres.

Ce mode de réalisation est particulièrement adapté aux cas où le composé d'intérêt est excrété par les cellules végétales puis diffuse ensuite hors des capsules. Ce mode de réalisation présente l'avantage de conserver intactes les capsules et les cellules.

Selon un autre mode de réalisation, la récupération du composé d'intérêt s'effectue par ouverture des capsules, puis par ouverture de la membrane des cellules, puis par traitement du mélange obtenu, par des méthodes de purification classiques.

L'ouverture des capsules peut être de type chimique ou mécanique. Un mode d'ouverture chimique est par exemple la dépolymérisation de la membrane, typiquement par mise en contact avec une solution d'ions citrate. Un mode d'ouverture mécanique est typiquement le broyage des capsules.

L'ouverture de la membrane des cellules est typiquement réalisée par broyage des cellules.

Ce mode de réalisation est particulièrement adapté aux cas où le composé d'intérêt reste confiné au sein des cellules végétales.

La présente invention a aussi pour objet l'utilisation d'une capsule selon l'invention, pour la production de cellules végétales et/ou la production de molécules d'intérêt.

Typiquement, la production de molécules d'intérêt est obtenue par élicitation de cellules végétales, telle que décrite ci-dessus.

### Composition

La présente invention a également pour objet une composition comprenant au moins une capsule selon l'invention.

Selon un mode de réalisation, la capsule selon l'invention a été traitée par un procédé de production d'un composé d'intérêt selon l'invention.

Selon une variante, la capsule selon l'invention a en outre subi un traitement postérieur au procédé de production d'un composé d'intérêt, consistant à former une seconde membrane encapsulant totalement à sa périphérie la membrane gélifiée de ladite capsule. Une telle seconde membrane peut être formée par gélification en présence d'un composé apte à former des liaisons électrostatiques avec les constituants de la membrane gélifiée, typiquement en présence d'un polyélectrolyte.

Cette variante présente l'avantage de protéger le cœur des capsules et d'éviter la migration hors des capsules des composés d'intérêt contenus dans ledit cœur.

La composition selon l'invention est typiquement une composition cosmétique, pharmaceutique ou agro-alimentaire.

La présente invention a aussi pour objet l'utilisation d'une capsule selon l'invention pour la préparation d'une composition cosmétique, pharmaceutique ou agro-alimentaire.

La présente invention a également pour objet une composition comprenant un extrait de capsule.

Par « extrait de capsule », on entend un composé d'intérêt produit par les cellules végétales, typiquement par élicitation, et récupéré comme décrit ci-dessus. On entend également une cellule végétale, issue d'un procédé de mise en culture de l'invention, et éventuellement issue d'un procédé de production d'un composé d'intérêt selon l'invention.

D'autres caractéristiques et avantages de l'invention ressortiront mieux des exemples qui suivent, donnés à titre illustratif et non limitatif.

### EXEMPLES

### Exemple 1 : Préparation de capsules gélifiées comprenant des micro-algues

### 1. Préparation des solutions pour la fabrication de capsules

### - Phase externe (membrane)

Une solution contenant 1,69% d'alginate de sodium *(Protanal LF200 FTS, FMC Bioploymer*) (w/w) et 1 mM (soit 0,0288% massique) de SDS (*Sigma Aldrich*) a été préparée, puis filtrée à 5 µm.

Cette étape de filtration permet d'éviter la présence de particules ou d'agrégats solides conduisant au bouchage des buses utilisées pour la production, mais est aussi utilisée pour stériliser les phases. Il est également possible de chauffer ces phases à une température supérieure à 60°C pour les stériliser.

### - Phase interne (cœur)

Une solution de micro-algues a été préparée à une concentration typique de 1 million de cellules/mL dans du milieu TAP (MC1).

De la 2-éthylcellulose (0,5% massique) a été ajoutée pour faciliter la co-extrusion des phases et stabiliser le procédé en évitant d'avoir de trop grandes différences de viscosité entre les phases internes et externes.

Les micro-algues suivantes ont notamment été encapsulées : *Chlamydomonas reinhardtii* (souches WTS24-, Sta6 et CW15).

### 2. Fabrication de capsules

Le procédé de fabrication de capsules est basé sur la co-extrusion concentrique de deux solutions, notamment décrite dans WO 2010/063937 et FR2964017, pour former des gouttes doubles.

La taille de la phase interne et l'épaisseur de la phase externe des gouttes formées ont été contrôlées par l'utilisation de deux pousse-seringues indépendants (HA PHD-2000).

Le ratio r_{q} entre le débit de fluide constituant le cœur et le débit de fluide constituant la membrane a été fixé à 1,6. Cela a permis d'obtenir des capsules ayant un ratio épaisseur de membrane sur rayon inférieur à 0,9, ce qui maximise le taux d'encapsulation de la phase interne, et donc des micro-algues.

Les capsules obtenues possèdent un diamètre de 300 µm (+/- 50 µm).

Les gouttes formées ont été gélifiées à l'aide d'une solution gélifiante de chlorure de calcium stérile à 200 mM (concentration minimale 50 mM), à laquelle ont été ajoutées quelques gouttes d'une solution stérile de Tween 20 (*Sigma Aldrich*) à 10% (w/w).

Les capsules formées ont été récoltées à l'aide d'un tamis, puis transvasées dans un des milieux de culture décrits ci-après.

### Exemple 2 : Production de micro-algues en capsules

### 1. Préparation des solutions

- *Tampon phosphate (2x) :*

| | |
|---|---|
| K₂HPO₄ | 14,34 g |
| KH₂PO₄ | 7,26 g |
| eau pure | 1 L |

- *Tampon phosphate 1M pH 7* :

| | |
|---|---|
| solution à 1 mol/L de K₂HPO₄ | 60 mL |
| solution à 1 mol/L de KH₂PO₄ | 40 mL |

- *Tampon Beijerincks (2x) :*

| | |
|---|---|
| NH₄Cl | 8 g |
| CaCl₂ | 1 g |
| MgSO₄ | 2 g |
| eau pure | 1 L |

- *Solution trace :*

| | | |
|---|---|---|
| Solution S1 | EDTA | 50 g |
| | eau pure | 250 mL |
| Solution S2 | ZnSO₄.7H₂O | 22 g |
| | eau pure | 100 mL |
| Solution S3 | H₃BO₃ | 11,4 g |
| | eau pure | 200 mL |
| Solution S4 | MnCl₂.4H₂O | 5,06 g |
| | FeSO₄.7H₂O | 4,99 g |
| | CoCm₂.6H₂O | 1,61 g |
| | CuSO₄.5H₂O | 1,57 g |
| | (NH₄)₆MO₇O₂.4H₂O | 1,1 g |
| | eau pure | 50 mL |

Pour préparer la Solution trace, les solutions S2, S3 et S4 ont été mélangées, puis la solution S1 a été ajoutée. Le mélange a été porté à ébullition pendant quelques minutes. Puis le mélange a été agité fortement en conservant la température au-dessus de 70°C. Le pH du mélange a ensuite été ajusté à une valeur comprise entre 6,5 et 6,8 par ajout de la quantité nécessaire d'une solution de KOH à 20% en masse. Le volume du mélange a ensuite été ajusté à 1 L par ajout d'eau pure. Le mélange a ensuite été laissé au repos durant une semaine, jusqu'à prendre une couleur rose/violette. La solution a enfin été filtrée.

### 2. Milieux de culture (MC2)

- *Milieu TAP :*

| | |
|---|---|
| Tris | 2,42 g |
| Beijerincks (2x) | 50 mL |
| Tampon phosphate 1M pH 7 | 1 mL |
| Solution trace | 1 mL |
| acide acétique | 1 mL |
| Eau pure | QSP 1 L |

- *Milieu Minimum :*

| | |
|---|---|
| Beijerincks (2x) | 50 mL |
| Tampon phosphate (2x) | 50 mL |
| Solution trace | 1 mL |
| Eau pure | QSP 1 L |

### 3. Culture et croissance de micro-algues encapsulées selon l'invention et de micro-algues en bulk

D'une part, les capsules de l'exemple 1, contenant des micro-algues *Chlamydomonas reinhardtii* (souche WTS 24-) selon une concentration cellulaire initiale ajustée à 1 million de cellules/mL, ont été mises en culture dans du milieu TAP ou du milieu Minimum (MC2) (soit environ 200 capsules par flasque de 20 mL, soit 10 000 capsules pour 1 L de milieu MC2), dans des flasques permettant les échanges gazeux, sous agitation modérée, à 25°C.

D'autre part, les mêmes micro-algues *Chlamydomonas reinhardtii* (souche WTS 24-) ont été mises en culture en *bulk,* selon une concentration cellulaire initiale ajustée à 1 million de cellules/mL, dans les mêmes conditions.

En milieu Minimum, une source de lumière de l'ordre de 3 400 lumen a été utilisée.

Le suivi de la croissance cellulaire a été fait de manière qualitative, en comparant l'évolution du volume occupé par les micro-algues dans les capsules, et de manière quantitative, par des expériences classiques de biologie cellulaire. Pour ce faire, les capsules ont été ouvertes par mise en contact avec une solution de citrate (de sodium) à 10% massique pendant quelques secondes. Les anions citrate permettent de complexer les cations calcium, ce qui dépolymérise le gel d'alginate de la membrane.

Le contenu des capsules a été analysé par cytométrie en flux et comptage Malassez.

Dans les capsules de l'invention, les micro-algues ont proliféré jusqu'à une concentration moyenne dans les capsules comprise entre 120 et 250 millions de cellules/mL au bout d'une semaine, telle que mesurée par comptage Malassez.

En comparaison, avec un procédé de culture en *bulk* (cellules non encapsulées, sous forme libre en suspension), les micro-algues n'ont pas dépassé une concentration de 10 millions de cellules/mL, toutes conditions égales par ailleurs.

### 4. Evaluation de la stabilité des capsules en milieu de culture (MC2)

La stabilité des capsules a été évaluée dans du milieu de culture TAP, du milieu de culture TAP additionné de 10 mM de CaCl₂ et du milieu de culture TAP auquel a été ajouté 0,1% d'EDTA en masse.

Les capsules de l'exemple 1 sont restées stables au moins 3 semaines dans chacun de ces 3 milieux de culture (MC2).

### 5. Evaluation de la protection mécanique apportée aux micro-algues par les capsules

Des micro-algues *Chlamydomonas reinhardtii* souche WTS 24- ont été mises en culture à 250 000 cellules/mL dans du milieu TAP (soit environ 10 000 capsules pour 1 L de milieu TAP), d'une part sous forme libre (en *bulk*), et d'autre part en version encapsulée selon l'invention (capsules de l'exemple 1).

Les deux échantillons obtenus ont été imagés en présence de SYTOX Green, un marqueur de mort cellulaire visible en fluorescence avec le filtre Nikon FITC, avant et après une forte agitation.

Avant agitation, on a observé que la plupart des micro-algues ne présentaient pas de marquage vert indicateur de mort cellulaire. Peu de cellules étaient donc mortes avant agitation, la proportion de micro-algues mortes correspondant simplement au cycle cellulaire.

Après agitation, on a observé que dans l'échantillon de micro-algues cultivées en *bulk,* la plupart des micro-algues apparaissaient colorées en vert, ce qui indique que la plupart des micro-algues sont mortes, sous l'effet du fort cisaillement provoqué par l'agitation.

Au contraire, les micro-algues encapsulées présentaient un marquage vert équivalent au marquage observé avant l'agitation. Peu de cellules sont mortes, malgré le fort cisaillement imposé. Comme dans la suspension de micro-algues initiale, la présence d'une faible proportion de micro-algues mortes est normale et découle simplement du cycle cellulaire des micro-algues considérées.

Une quantification globale de la fluorescence associée à la mort cellulaire a été réalisée sur chaque échantillon après agitation et a montré que la mort cellulaire était considérablement plus élevée dans le cas des micro-algues en *bulk,* que dans le cas des micro-algues encapsulées.

Cette expérience a mis en évidence la protection mécanique conférée par les capsules aux micro-algues.

### 6. Semi-perméabilité des membranes : molécules VS bactéries

Des capsules selon l'exemple 1 ont été mises en culture dans du milieu TAP (soit environ 10 000 capsules pour 1L de milieu TAP), auquel ont été ajoutées des bactéries *Escherichia coli* RFP, c'est-à-dire des bactéries de l'espèce *E. coli* génétiquement modifiées pour qu'elles synthétisent une molécule fluorescente, visible en utilisant le filtre Nikon TRITC.

Il a été constaté que les bactéries RFP n'envahissaient pas l'intérieur des capsules.

D'autre part, des capsules selon l'exemple 1 ont été mises en présence d'une solution à 1 mM de rhodamine (visible en fluorescence en utilisant le filtre Nikon TRITC) pendant quelques minutes, puis ont été transférées dans un bain d'huile et imagées au microscope. Ces résultats ont montré que, contrairement aux bactéries *E. coli* RFP, la rhodamine a diffusé à travers la membrane d'alginate pour se retrouver à l'intérieur des capsules.

Les capsules selon l'invention sont donc semi-perméables : elles permettent le passage de molécules, tels que les nutriments du milieu de culture MC2, et ne laissent pas pénétrer les bactéries, ce qui a pour avantage d'empêcher toute contamination bactérienne pendant le procédé de culture des micro-algues.

### EXEMPLE 3 : Elicitation de micro-algues encapsulées pour la production de lipides et de sélénium organique

### 1. Production de lipides

Le tampon Beijerincks 2X N0 a été préparée :

| | |
|---|---|
| CaCl₂ | 1 g |
| MgSO₄ | 2 g |
| eau pure | 1 L |

Le milieu N0 a été préparé :

| | |
|---|---|
| Tris | 2,42 g |
| Beijerincks (2x) N0 | 50 mL |
| Tampon phosphate 1M pH 7 | 1 mL |
| Solution trace | 1 mL |
| acide acétique | 1 mL |
| Eau pure | QSP 1 L |

Des capsules selon l'exemple 1, comprenant des micro-algues *Chlamydomonas reinhardtii* (souche Sta6) ont été mises en culture dans du milieu TAP pendant 48 h (soit environ 10 000 capsules pour 1L de milieu TAP), puis ce dernier a été éliminé et remplacé par du milieu N0, pendant 48 h.

Les micro-algues ont alors produit des lipides, sous forme de corps lipidiques présents à l'intérieur desdites micro-algues.

Des capsules ont été prélevées et mises en présence de 25% de DMSO et 1 µM de Nile Red pendant 10 minutes. Les capsules ont ensuite été imagées au microscope en champ clair et en fluorescence (source : lampe à mercure).

Le chloroplaste des micro-algues a été révélé en fluorescence grâce au filtre Nikon UV-1A.

Le Nile Red, témoignant de la présence lipides produits par élicitation, a été révélé grâce au filtre Nikon FITC.

### 2. Production de sélénium organique

Certaines micro-algues produisent des molécules contenant du sélénium, comme par exemple *Peridinium cinctum.* De telles micro-algues peuvent donc produire du sélénium sous forme organique, à partir de sélénium minéral.

Des micro-algues produisant des molécules contenant du sélénium ont été encapsulées conformément à la présente invention, puis mises en incubation dans un milieu enrichi en sélénium minéral, afin d'induire une bioaccumulation de sélénium organique par ces micro-algues.

La quantification de Sélénium bio-accumulé par les micro-algues encapsulées a été réalisée par extraction des cellules des capsules, puis par l'utilisation de méthodes physiques, comme l'AAS (cf. Niedzielski (2002), Polish Journal of Environmental Studies : « Atomic Absorption Spectrometry in Détermination of Arsenic, Antimony and Selenium in Environmental Samples »), ou par l'utilisation d'un bioassay (cf. Lindstrôm (1983), Hydrobiologia : « Selenium as a growth factor for plankton algae in laboratory experiments and in some Swedish lakes »).

## Revendications

1. Capsule comprenant :
- une phase interne comprenant au moins une cellule végétale et au moins un agent de viscosité, et
- une phase externe gélifiée, encapsulant totalement ladite phase interne à sa périphérie, ladite phase externe comprenant au moins un tensioactif et au moins un polyélectrolyte à l'état gélifié.

2. Capsule selon la revendication 1, dans laquelle la phase interne comprend un milieu de culture.

3. Capsule selon la revendication 1 ou 2, dans laquelle la ou les cellules végétales sont des cellules d'algues, de préférence des cellules de micro-algues.

4. Capsule selon l'une quelconque des revendications 1 à 3, dans laquelle la ou les cellules végétales sont en suspension dans la phase interne.

5. Capsule selon l'une des revendications 1 à 4, dans laquelle le polyélectrolyte est un polyélectrolyte réactif aux ions multivalents, de préférence un alginate.

6. Capsule selon l'une des revendications 1 à 4, **caractérisée en ce qu'**elle présente une taille inférieure à 5 mm, de préférence comprise de 50 µm à 3 mm.

7. Procédé de culture de cellules végétales comprenant une étape de mise en culture d'au moins une capsule selon l'une des revendications 1 à 6.

8. Procédé de production d'un composé d'intérêt, ledit procédé comprenant :
- une étape de mise en culture d'au moins une capsule selon l'une des revendications 1 à 6, ladite capsule comprenant au moins une cellule végétale apte à produire ledit composé d'intérêt,
- éventuellement, une étape d'élicitation des cellules végétales comprises dans ladite capsule, et
- une étape de récupération du composé d'intérêt.

9. Utilisation d'une capsule selon l'une quelconque des revendications 1 à 6, pour la production de cellules végétales et/ou la production de composés d'intérêt.

10. Composition comprenant au moins une capsule selon l'une des revendications 1 à 6.
